# EUROPEAN PATENT SPECIFICATION

(11) **EP 0 594 739 B1**
(45) Date of publication and mention of the grant of the patent: **01.03.2000**
(21) Application number: 92915667.7
(22) Date of filing: 13.07.1992
(51) Int. Cl.: A61K 51/00

(54) **CANCER TREATMENT**
BEHANDLUNGSVERFAHREN FUER KREBS
TRAITEMENT DU CANCER

(30) Priority: 12.07.1991 GB 9115192
(43) Date of publication of application: 04.05.1994
(73) Proprietor: ANTISOMA RESEARCH LIMITED, London W5 3QR (GB)
(72) Inventor: STEWART, John, Simon, Watson, London W4 4EG (GB)
(74) Representative: Thomas, Philip John Duval
(86) International application number: GB9201280
(87) International publication number: WO9300927

(56) References cited:
- EP-A- 0 035 265
- EP-A- 0 198 257
- British Journal of Cancer, vol. 63, no. 1, January 1991, M. GERRETSEN et al.: "Superior localisation and imaging of radiolabelled monoclonal antibody E48 F(ab')2 fragment in xenografts of human squamous cell carcinoma of the head and neck and of the vulva as compared to monoclonal antibody E48 IgG", pages 37-44, see entire document, especially: "Discussion"
- International Journal of Cancer, vol. 44, no. 3, 15 September 1989, Alan R. Liss, Inc., J.J. QUAK et al.: "Localization and imaging of radiolabelled monoclonal antibody against squamous-cell carcinoma of the head and neck in tumor-bearing nude mice", pages 534-538, see entire document
- MOYLAN ET AL [1985] CANCER TREATMENT SYMPOSIA VOL.2, pp 109-113
- ORDER ET AL [1985] J.CLIN.OCOL., VOL.3, No.2, pp 1537-1582

## Description

The present invention relates to the treatment of cancer, and especially to the treatment of head or neck squamous cell carcinoma (SCC).

Head and neck squamous cell carcinoma (SCC) often presents at a late stage with extensive locally advanced disease, or, following histological examination, a seemingly completely resected lesion proves to have infiltrated surgical margins. Treatment of these patients is difficult and often controversial. Despite radical surgery with lymph node dissection and optimal external beam irradiation, only approximately 30% of these patients achieve a 5 year survival. Most patients die from local recurrence of their tumour, often in the irradiated field, in the draining lymph nodes or at the surgical borders¹. There is ample experimental evidence indicating that, were it possible to administer an extra 1000-2000 cGy to the treated site, there would be a significant increase in the number of patients achieving better tumour control and prolonged survival due to enhanced control of microscopic disease¹. However, toxicity to normal tissues (for example skin breakdown, cervical myelopathy or cranial nerve palsies) precludes dose escalation above the currently administered 7000 cGy.

There are also problems with administering high doses of external radiation to patients with cancer of the cervix, bladder, bowel, rectum and bronchus.

The production of monoclonal antibodies (MAbs) through the fusion technique has revolutionised many areas of experimental, clinical and industrial work. Since the first pioneering work of Köhler and Milstein, MAbs have found application in every aspect of biomedical research. They are currently used as probes into the fine structure of cellular components, in histocompatibility tests, radioimmunoassays, molecule purification procedures, immunohistochemistry, flow cytometry and electron microscopy. MAbs have provided new approaches to many problems in oncology. In addition to improving the detection and localisation of tumours *in vivo* either through sensitive serum assays or through radioimmunoscintigraphy, they have improved the grading, staging and classification of haemopoietic malignancies and may provide significant prognostic information in a variety of tumours. Finally, MAbs have been suggested for use in novel therapeutic approaches, using their potential ability to target tumour cells. The use of MAbs unmodified or as vectors for radioisotopes, cytotoxic agents, enzymes or toxins to selectively target and kill tumour cells is the subject of much current research.

Some of the prerequisites for a MAb-radioisotope conjugate to be therapeutically effective are operational specificity, capacity to target a well characterised surface antigen, high affinity for the antigen, reaction with the majority of tumour cells and adequate stability *in vivo*. Trials are currently underway to investigate the efficacy of MAb-mediated radioimmunotherapy, and encouraging preliminary results have been obtained in a variety of tumours². Nevertheless several problems have emerged, namely low MAb uptake by the tumour, dissociation of the isotope from the antibody *in vivo*, toxicity to vital organs, human antimouse (HAMA) responses, circulating antigen resulting in Ab-Ag complex formation and low amounts of antibody reaching the tumour via the intravenous route³. Increasingly sophisticated techniques are being devised, for example the production of bispecific, chimeric, single chain, human or even single domain antibodies. Furthermore two-step avidin-biotin schemes⁴, the use of improved chelating agents and linkers and regional (intracavitary, intrathecal, intraperitoneal and intraarterial) routes of therapy instead of the usual i.v. route have shown promising results⁵. The major limitation of intravenously-administered radioimmunotherapy is the low absolute amount of mab reaching the tumour. The data obtained in several studies indicate that the maximum tumour irradiation dose that can be achieved with tolerable toxicity is 1500-2000 Cgy, which is well below tumouricidal levels for most human tumours. The causes are far from clear, but physiological protection mechanisms in the tumour (eg dehalogenation enzymes), poor tumour vascularisation and low penetration of the antibody seem to play a major role.

Moylan *et al* (1985) Cancer Treatment Symposia Vol 2, 109-113 relates to the use of radiolabelled antibody as an adjuvant to external beam radiation therapy in a study to determine the toxic effects of polyclonal ¹³¹I antiferritin and anti-CEA. Page 111 mentions administration of the radiolabelled antibody 3 weeks after the completion of external beam radiotherapy.

Order *et al* (1985) *J. Clin. Oncol* 3, No. 12, pp 1537-1582 relates to the treatment of hematoma using ¹³¹I antiferritin which is administered at least one month after external beam irradiation. On page 1579 the authors warn of the dangers of using a combination of external radiation and chemotherapy, especially reduction of marrow reserve and conclude that they would not use such an approach in future protocols.

We have now found that a combination of externally-applied radiation and radioimmunotherapy is beneficial in the treatment of cancers.

A method of treating a cancer in a mammal, comprising administering a preparation carrying a cytotoxic level of radioactive atoms, the preparation being specific for the cancer cells, and in conjunction externally administering a cytotoxic dose of radiation to the cancer, is described.

One aspect of the invention provides the use of a preparation specific for cancer cells and carrying a cytotoxic level of radioactive atoms in the manufacture of a medicament for the treatment of cancer of a mammal in conjunction with an externally administered cytotoxic dose of radiation, as defined in the accompanying claims.

The cancer is preferably squamous cell carcinoma of the head or neck. It may, however, be of any solid tumour, for example cancer of the cervix, bladder, bowel, rectum or bronchus.

The preparation is preferably a preparation of antibodies but may be any compound specific for the cancer cells, for example a ligand specific for a cell-surface receptor preferentially expressed by cancer cells. By "antibody" we mean any molecule which retains a useful level of the cancer specificity of a cancer-specific immunoglobulin compound and of which the specificity-conferring region or regions is or are at least 50% homologous (preferably at least 80%, 90%, 95% or 99% homologous) to the most similar specificity-conferring region or regions of the said immunoglobulin. The antibody preparation may be a polyclonal collection of antibodies of restricted specificity. Preferably the antibody is a monoclonal antibody, an F_{ab}', (F_{ab})₂, Fᵥ, scFᵥ or dAb fragment or a synthetic molecule including the minimum recognition unit(s) of a cancer-specific immunoglobulin. Such immunoglobulins may be prepared by conventional methods for preparing monoclonal antibodies (see Monoclonal Hybridoma Antibodies Ed. J.G.R. Hurrell, CRC Press 1982, incorporated herein by reference) using cancer-associated antigens, for example epidermal growth factor receptors and polymorphic epithelial mucins.

By "specific", we mean that it is possible to achieve a useful difference between the association of the targeting means with the cancer cells and the association of the targeting means with other cells; complete non-association with normal cells is not required. A tumour:mucosa ratio of at least 4:1, preferably at least 5:1 or 10:1 is desirable.

Non-antibody targeting means include epidermal growth factor and homologues and fragments thereof (for cancers such as squamous cell carcinoma of the head or neck which express high levels of the EGF receptor), urokinase-type plasminogen activator and receptor-binding portions thereof, melanocyte-stimulating hormone and fragments thereof, etc.

The cytotoxic radioactive atom may, for example, be iodine-131, iodine-123, iodine-125, rhenium-186, rhenium-188 or yttrium-90. Such atoms may be attached to the cancer-specific compound by conventional techniques, for example via a cysteine residue (iodine or rhenium) or lysine residue (yttrium) or using the IODOGEN method (Fraker, P.J. *et al* (1978) *Biochem. Biophys. Res. Commun*. **80**, 49-57). Other methods are described in detail in "Monoclonal Antibodies in Immunoscintigraphy", J-F Chatal (CRC Press 1989).

Preferably, sufficient radioactive atom is administered for a radiation dose of at least 500 cGy to be delivered to the tumour, more preferably at least 1000 cGy or at least 1500 cGy. A dose of more than 3000 cGy may be undesirably toxic or difficult to administer, and a maximum of 2000 cGy is preferred. The cancer-specific preparation is made up conventionally, typically in pyrogen-free, sterile, saline, typically for intravenous injection, as is known.

By "administering in conjunction with", we mean that the radioimmunotherapy and the externally applied radiation are administered sufficiently closely in time for the beneficial effect on the tumours to be greater than if the two forms of treatment are applied with a gap of 3 weeks between them. Preferably, the administration of the cancer-specific preparation takes place during the administration of the externally applied radiation. Thus, in a typical 5 week course of daily irradiation, the radioactive cancer-specific preparation is administered on about day 10-20, preferably at about day 15. However, the cancer-specific preparation can be administered up to about 2 weeks before external irradiation begins or up to about 2 weeks after external irradiation finishes.

The external radiation may be delivered in a conventional way, for example using a linear accelerator, to deliver a dose of about 1000-7000 cGy, preferably about 5000 cGy, over a 1-10 week period, preferably about 4-6 weeks, in daily (Monday-Friday) doses.

Suitably, the patient has already undergone surgery to remove most of the tumour, and the methods and compositions of the invention are used to further improve the chances of success. Preferably, the amount of tumour present at the time of treatment does not exceed 100 g, more preferably it does not exceed 20 g, more preferably still it does not exceed 10 g and ideally it does not exceed 5 g. Preferably, it is less than 5 cm in diameter, more preferably less than 2 cm in diameter and ideally less than 1 cm in diameter. If the tumour is detected when it is so small, as can be the case with cancer of the cervix then the methods of the invention may be usefully employed without the necessity of surgical intervention.

Preferred embodiments of the invention will now be described by way of example.

### Example 1

The first objective is to choose a suitable MAb against a SCC antigen for *in vivo* use. Immunohistochemistry may be performed on snap-frozen and paraffin-embedded tumour samples with a panel of MAbs against SCC antigens. Tumour xenografts may be established in nude athymic mice using SCC cell lines and the kinetics of the MAbs assessed. Immunohistology is performed on the xenografts, and macro- and micro-biodistribution studied.

### Methods

### 1. Immunohistology

The two-step immunoperoxidase method using a primary mouse MAb and a second layer rabbit antimouse immunoglobulin is used. The reaction is developed with 3,3-diaminobenzidine tetrachloride (DAB) substrate. Suitable controls are used.

### 2. Cell Cultures

SCC cell lines are cultured at 37°C in a humidified atmosphere of 5% CO₂ either in RPMI-1640 + 10% FCS or in DMEM + 10% FCS according to each cell line's requirements.

### 3. Xenografts

Tumours are established by subcutaneous injection of 5 x 10⁶ cells in 100 µl tissue culture medium in the right flank of female athymic nude mice.

### 4. Radiolabelling

### Iodination

Antibodies are labelled with ¹²³I, ¹³¹I or ¹²⁵I using the iodogen technique. The radiolabelled antibody is purified by gel filtration on a 20 ml Sephadex G-50 column previously treated with HSA.

### Example 2

A 200 mCi dose of ¹³¹I-labelled anti-(CA larynx) MAb (ie a MAb against a carcinoma of the larynx or tongue) is used, to give a tumour uptake of about 0.0002 mCi of ¹³¹I per gram of tumour. The total dose to the tumour is about 60 Gy, with a whole body radiation dose (WBRD) of about 15 Gy. The required additional 20 Gy is supplied by external beam radiation.

The following tables detail the dose rates for:
1) macroscopic tumour deposits in a small body volume
2) microscopic tumour deposits in a large body volume

| **Eradication of macroscopic tumour deposits in a small body volume (700 cm**^{**3**}**)** | | | |
|---|---|---|---|
| | External Beam | MAb | Total |
| Tumour Cells | 65 Gy | 20 Gy | 85 Gy |
| Normal Cells | 65 Gy | 5 Gy | 70 Gy |
| Whole Body | 0 Gy | 5 Gy | 5 Gy |
| Kidney | 0 Gy | 15 Gy | 15 Gy |
| Liver | 0 Gy | 20 Gy | 20 Gy |

### Indium-111

The antibody is conjugated to the chelating agent DTPA, using the bicyclic anhydride method⁶. Free DTPA is separated from DTPA-MAb by Sephadex G-50 gel-filtration using 0.1 M sodium acetate as elution buffer. ¹¹¹In citrate is added to the DTPA conjugate and allowed to react for 15-30 mins. Purification is as for the iodinated MAb.

### Yttrium-90

Yttrium-90 in 0.04 M hydrochloric acid is complexed using 0.5 sodium acetate. DTPA-conjugated antibody is added to the complex. After 1-2 h incubation, EDTA is added to react with free ⁹⁰Y Separation is as for iodinated MAb.

### 5. Biodistribution

The MAbs are administered intravenously to unanaesthetised mice. Dual labelling experiments may be performed using ¹³¹I and ¹²⁵I to compare antibody kinetics in the same animal. At fixed intervals after injection, groups of mice are sacrificed and tumour, normal tissue and blood obtained, weighed and the radioactivity counted in a γ-counter, along with serially diluted standards of injectate. Results may be expressed as percentage of injected dose per gram of wet tissue (%i.d./g) and as tumour to normal tissue ratios. The specificity index (S.I.) for a tissue may be calculated. The higher the S.I. of a tissue the more specific the antibody is for the tumour than that tissue.

### 6. Autoradiography

Gross autoradiography is performed by placing histological slides in direct contact with autoradiographic film. Exposure time is 8 days at 4°C. Exposed films are developed for 5 min, fixed and dried. For micro-autoradiography, sections are placed on gelatin coated slides, dried at 37°C overnight, hydrated and dipped in Ilford K5 emulsion (1:1 dilution). Exposure time is 3 weeks at 4°C in light-proof boxes. Developing time is 3-5 min.

Studying the kinetics and biodistribution of the MAbs allows the most promising MAbs to be selected for use in patients. These MAbs are radiolabelled and injected intravenously prior to surgery. Radioimmunoscintigraphy is carried out including anterior, posterior and spot views⁷. Tumour and normal tissue samples are obtained at surgery. Biodistribution studies are carried out as described above. Blood and urine samples are serially obtained and aliquots counted in the γ-counter, along with standard injectate dilutions. Following centrifugation, radioactivity is measured in the plasma and the cell pellet.

The final stage is to administer a MAb selected from the biodistribution and imaging studies as a radiation delivery vehicle in patients with head and neck carcinoma, in conjunction with externally applied radiation. A dose of 5000 cGy is applied as 25 daily (Monday-Friday) doses of 200 cGy over 5 weeks and 2000 cGy of radioactive antibody is administered as a single i.v. dose on day 15.

### References

1. DeVita, V.T. *et al* (1989) Cancer: Principles and Practice of Oncology **1&2** Philadelphia: J.B. Lipincott Company.
2. Order, S.E. (1989) Therapeutic use of radioimmunoconjugates, antibody immunoconjugates and radiopharmaceuticals **2: No 4,** 235-239.
3. Epenetos, A.A. *et al* (1986) Limitations of radiolabelled monoclonal antibodies for localisation of human neoplasms. *Cancer Res.* **46:** 3183-3192.
4. Paganelli, G*. et al* (1988) *In vivo* labelling of biotinylated monoclonal antibodies by radioactive avidin. A strategy to increase tumour radiolocalisation. *Int. J. Cancer Suppl*. **2:** 121-125.
5. Hooker, G.R & Epenetos, A.A. (1986) The intracavitary application of monoclonal antibodies as vehicles for therapeutic radionucleotides P.A. Schubiger and P.H. Hassler (eds) Radionuclides for therapy: 183-199 Roche Scientific Service Publications Basel.
6. Hnatowich, D.J. *et al* (1983) The preparation of DTPA-coupled antibodies radiolabelled with metallic radionucleotides: an improved method. *J. Immunol. Methods* **65:** 147-157.
7. Soo, K.C. *et al* (1987) Radioimmunoscintigraphy of squamous carcinomas of the Head and Neck. *Head and Neck Surgery* **9:** 349-352.

## Claims

1. The use of a preparation specific for cancer cells and carrying a cytotoxic level of radioactive atoms in the manufacture of a medicament for treatment of cancer of a mammal; characterised in that the cancer-specific preparation is administered in conjunction with an externally administered cytotoxic dose of radiation; in that the cancer-specific preparation is administered up to about 2 weeks before external irradiation begins or up to about 2 weeks after external irradiation finishes; and in that the cancer-specific preparation and external irradiation are administered sufficiently closely in time for the beneficial effect on the tumours to be greater than if the two forms of treatment are applied with a gap of 3 weeks between them.

2. The use according to Claim 1 wherein the cancer is squamous cell carcinoma (SCC) of the head or neck.

3. The use according to Claim 1 or 2 wherein the said preparation is a preparation of antibodies.

4. The use according to Claim 1, 2 or 3 wherein the cytotoxic radioactive atom is iodine-131, iodine-123, iodine-125, rhenium-186, rhenium-188 or yttrium-90.

5. The use according to any one of the preceding claims wherein the medicament is suitable for administration of a radiation dose of 500-2000 cGy to be delivered to the tumour by the radioactive atoms.

6. The use according to any one of the preceding claims wherein the cancer-specific preparation is suitable for administration during a course of external radiation.

7. The use according to any one of the preceding claims wherein the mammal has already undergone surgery to remove most of the cancer.

8. The use according to any one of the preceding claims wherein the amount of tumour present at the time of treatment does not exceed 10 g.

9. The use as claimed in any one of Claims 1 or 3 to 8 wherein the cancer is any solid tumour.

10. The use as claimed in Claim 9 wherein the cancer is of the cervix, bladder, bowel, rectum or bronchus.

## Patentansprüche

1. Verwendung eines für Krebszellen spezifischen und eine cytotoxische Menge radioaktiver Atome tragenden Präparats bei der Herstellung eines Medikaments zur Behandlung von Krebs bei einem Säugetier, dadurch gekennzeichnet, daß das krebsspezifische Präparat in Verbindung mit einer extern verabreichten cytotoxischen Strahlendosis verabreicht wird, das krebsspezifische Präparat bis zu etwa 2 Wochen vor Beginn der externen Bestrahlung oder bis zu etwa 2 Wochen nach Beendigung der externen Bestrahlung verabreicht wird und das krebsspezifische Präparat und die externe Bestrahlung zeitlich ausreichend eng beieinander verabreicht werden, damit der Nutzeffekt auf die Tumore stärker ist als bei Applikation der beiden Behandlungsformen mit einem Zwischenraum von 3 Wochen zwischen ihnen.

2. Verwendung nach Anspruch 1, wobei es sich bei dem Krebs um ein squamöses Zellkarzinom (SCC) des Kopfs oder Halses handelt.

3. Verwendung nach Anspruch 1 oder 2, wobei es sich bei dem Präparat um ein Antikörperpräparat handelt.

4. Verwendung nach Anspruch 1, 2 oder 3, wobei es sich bei dem cytotoxischen radioaktiven Atom um Iod-131, Iod-123, Iod-125, Rhenium-186, Rhenium-188 oder Yttrium-90 handelt.

5. Verwendung nach einem der vorhergehenden Ansprüche, wobei das Medikament zur Verabreichung einer Strahlendosis von an den Tumor durch die radioaktiven Atome abzugebenden 500-2000 cGy geeignet ist.

6. Verwendung nach einem der vorhergehenden Ansprüche, wobei das krebsspezifische Präparat zur Verabreichung im Laufe einer externen Bestrahlung geeignet ist.

7. Verwendung nach einem der vorhergehenden Ansprüche, wobei das Säugetier bereits zur Entfernung des Hauptteils des Krebses einer chirurgischen Behandlung unterworfen wurde.

8. Verwendung nach einem der vorhergehenden Ansprüche, wobei die Menge des zum Behandlungszeitpunkt vorhandenen Tumors 10 g nicht übersteigt.

9. Verwendung nach einem der Ansprüche 1 oder 3 bis 8, wobei es sich bei dem Krebs um irgendeinen festen Tumor handelt.

10. Verwendung nach Anspruch 9, wobei es sich bei dem Krebs um Gebärmutterhals-, Blasen-, Darm-, Rektum- oder Bronchialkrebs handelt.

## Revendications

1. Utilisation d'une préparation spécifique pour des cellules cancéreuses et présentant une concentration cytotoxique d'atomes radioactifs, dans la fabrication d'un médicament pour le traitement du cancer d'un mammifère ; caractérisée en ce que la préparation spécifique au cancer est administrée conjointement avec une dose cytotoxique de rayonnement administrée de l'extérieur ; en ce que la préparation spécifique au cancer est administrée jusqu'à environ 2 semaines avant le début de l'irradiation externe ou jusqu'à environ 2 semaines après la fin de l'irradiation externe ; et en ce que la préparation spécifique au cancer et l'irradiation externe sont administrées dans un temps suffisamment rapproché pour que l'effet bénéfique sur les tumeurs soit plus grand que si les deux formes de traitement sont appliquées à un intervalle de 3 semaines entre elles.

2. Utilisation selon la revendication 1, dans laquelle le cancer est un carcinome spinocellulaire (SCC) de la tête ou du cou.

3. Utilisation selon la revendication 1 ou 2, dans laquelle ladite préparation est une préparation d'anticorps.

4. Utilisation selon la revendication 1, 2 ou 3, dans laquelle l'atome radioactif cytotoxique est l'iode-131, l'iode-123, l'iode-125, le rhénium-186, le rhénium 188 ou l'yttrium-90.

5. Utilisation selon l'une quelconque des revendications précédentes, dans laquelle le médicament est approprié pour l'administration d'une dose de rayonnement de 500 à 2000 cGy devant être délivrée à la tumeur par les atomes radioactifs.

6. Utilisation selon l'une quelconque des revendications précédentes, dans laquelle la préparation spécifique au cancer est appropriée pour l'administration au cours d'un rayonnement externe.

7. Utilisation selon l'une quelconque des revendications précédentes, dans laquelle le mammifère a déjà subi une opération chirurgicale pour éliminer la plus grande partie du cancer.

8. Utilisation selon l'une quelconque des revendications précédentes, dans laquelle la quantité de tumeur présente au moment du traitement ne dépasse pas 10 g.

9. Utilisation selon l'une quelconque des revendications 1 ou 3 à 8, dans laquelle le cancer est toute tumeur solide.

10. Utilisation selon la revendication 9, dans laquelle le cancer est du col, de la vessie, de l'intestin, du rectum ou des bronches.
